Europäisches Patentamt

European Patent Office

Office européen des brevets

(19)

(11) Publication number: **0 074 240 B1**

## EUROPEAN PATENT SPECIFICATION

(12)

(45) Date of publication of patent specification: **20.03.91**

(51) Int. Cl.⁵: **A61K 39/40**

(21) Application number: **82304579.4**

(22) Date of filing: **31.08.82**

The file contains technical information submitted after the application was filed and not included in this specification

(54) Antibodies specific to endotoxins.

(30) Priority: **31.08.81 ZA 816020**

(43) Date of publication of application:
**16.03.83 Bulletin 83/11**

(45) Publication of the grant of the patent:
**20.03.91 Bulletin 91/12**

(34) Designated Contracting States:
**AT BE CH DE FR GB IT LI LU NL SE**

(56) References cited:
**US-A- 3 626 057**
**US-A- 4 027 010**

**BIOLOGICAL ABSTRACTS, vol. 63, 15th March 1977, no. 33543; NG, AH-KAU et al.: "Relationship of structure to function in bacterial endotoxins: Serologically cross-reactive components and their effect on protection of mice against some gram-negative infections" & J GEN MICROBIOL 94(1): 107-116. 1976**

(73) Proprietor: **Gaffin, Stephen Leslie**
**15 Sport Road**
**Durban Natal(ZA)**

(72) Inventor: **Gaffin, Stephen Leslie**
**15 Sport Road**
**Durban Natal(ZA)**

(74) Representative: **Weinzinger, Arnulf, Dipl.-Ing. et al**
**Patentanwälte Dipl.-Ing. Helmut Sonn Dr. Heinrich Pawloy Dipl.-Ing. Arnulf Weinzinger**
**Riemergasse 14**
**A-1010 Wien(AT)**

BIOLOGICAL ABSTRACTS, vol. 61, 15th January 1976, no. 7889; ANN-MARI SVEN-NERHOLM: "Experimental studies on cholera immunization: 4. The antibody response to formalized vibrio cholerae and purified endotoxin with special reference to protective capacity" & INT ARCH ALLERGY APPL IMMUNOL 49(4): 434-452. 1975

BIOLOGICAL ABSTRACTS, vol. 62, 15th November 1976, no. 54897; HILL, ALAN W. et al.: "Increased antibacterial activity against Escherichia coli in bovine serum after the infection of endotoxin tolerance" & INFECT IMMUN 14(1) 257-265. 1976

CHEMICAL ABSTRACTS, vol. 75, no. 9, 30th August 1971, page 261, no. 61443s, Columbus, Ohio, US; A.T.BALL et al.: "Humoral antibody response in rabbits after intragingival injections of bovine serum albumin and Escherichia coli endotoxin" & ARCH. ORAL BIOL. 1971, 16(3), 247-57

## Description

This invention relates to compositions of antibodies specific to endotoxins.

Gram negative bacteremia and endotoxemia is an important medical problem carrying a mortality of 30-80 % and causes millions of deaths in humans and animals world wide every year. Conventional antibiotic therapy is becoming less successful with time because antibiotics kill bacteria but do not at all reduce the amount of the extremely potent and stable, endotoxin released from gram negative bacterial cells. This endotoxin is still active upon death of bacteria and can continue to cause shock and death. In addition under certain conditions endotoxin may leak out of the intestine where it is always present and enter the systemic blood causing shock and death. A notable result of this is the very high mortality of farm animals having gram negative sepsis.

Endotoxin is known to be present in the intestines and under certain conditions of stress may leak out through the intestinal wall and enter the systemic blood supply, overwhelming the liver's detoxifying capacity and causing shock and death. In these cases there may not have been a stage of bacteremia prior to endotoxemia.

A means of inactivating or reducing the free concentration of endotoxins is by administering appropriate antibodies specific to endotoxins.

In the prior art it is known to immunise cows with the "pili" of gram negative bacteria to produce milk rich in "antipili" antibodies. Such antibodies prevent the attachment or adhesion of gram negative bacteria to the mucosal surface in the gut and in this manner reduce bacterial infection.

In Experimental Studies on Cholera Immunization (Int. Archs. Allergy appl. Immun. 49 :434-452 (1975) Ann-Marie Svennerholm describes a study to investigate the antibody response in rabbits after immunization with formalin-killed vibrios or cholera lipopolysaccharide (LPS) with regard to amount, immunoglobulin class and binding qualities of the antibodies induced. The significance of these parameters and of different immunization schedules for the protective capacity against experimental cholera in rabbits of immune sera and of purified anti-LPS antibodies was analyzed. The study was concerned with cholera only.

Elizabeth J. Ziegler et al (the Journal of Immunology Vol. III. No. 2. August 1973, 433-438) describes the immunization of rabbits with a UDP-galactose-deficient mutant (J5) of Escherichia coli 0111 that is unencumbered by 0 antigen since it cannot incorporate galactose into core LPS. Without 0 side chains, the incomplete lipopolysaccharide, the so-called core glycolipid, is accessible for producing antiserum to a wide range of bacteria with similar cores. In practice, however, the polysaccharide barrier has to be penetrated by the resulting anti-endotoxins before they can be effective and this has been a limiting factor against the use of such compositions. The method described in this document uses whole killed organisms as antigens.

It is an object of the invention to provide a composition containing a high titre of antibodies which are specific to endotoxins produced by gram negative bacteria. It is a further object of the invention to incorporate such antibodies in products for human and veterinary use.

The present invention provides a composition comprising antibodies produced in response to a mixture of endotoxins obtained from Salmonella enteriditis, Salmonella typhosa, Salmonella typhimurium, Salmonella abortus equii, Escherichia coli 026:B6, Escherichia coli 0128:B12, Escherichia coli 0127:B8, Escherichia coli 055:B5, Salmonella minnesota, Serratia marcescens, and Klebsiella pneumoniae, and, optionally, in addition from Shigella flexneri and or E. coli 0111.B4; or from Salmonella typhosa, Salmonella typhimurium, Salmonella abortus equii, Shigella flexneri, Escherichia coli 055:B5, Escherichia coli 0127:B8, Escherichia coli 0128:B12, Escherichia coli 026:B6, Escherichia coli 0111:B4, Salmonella minnesota, Serratia marcescens and Klebsiella pneumoniae, generally in admixture with a carrier medium. The carrier medium preferably comprises serum obtained by suitable treatment of the blood of animals or it may be synthesised from suitable materials; for example, it may be physiological saline. The composition may be in a form suitable for oral or parenteral administration to animals and or humans, or for incorporation in milk or a milk derivative.

The antibodies are specific to endotoxin(s) produced by gram negative bacteria of more than one of the following genera: Salmonella, Eschericia, Pseudomonas, Shigella, Serratia, Vibrio and Yersinia. It will be appreciated that an antibody or antibodies may react with the endotoxin-producing organism itself, in addition to "free" endotoxin(s) produced by the organism. Such binding to the endotoxin coat of living bacteria may activate complement which in turn may cause lysis and death of the bacteria.

According to a further aspect, the invention provides a method of producing a composition as specified above, which process includes the steps of inoculating a suitable animal with an antigen mixture capable of triggering the appropriate immunological response in the animal and removing a body fluid containing the resulting antibody or mixture of antibodies. The body fluid may be worked up in a known manner to give a preparation suitable for therapeutic and or prophylactic use in a human and/or animal. Such methods are well known in the art.

The present invention further provides milk or serum comprising a mixture of antibodies obtained from an animal that has been inoculated with endotoxins obtained from bacteria as defined above, suitable for use in producing a composition of the present invention, that is to say, by working up in a suitable manner.

The invention also provides a mixture of endotoxins obtained from bacteria as defined above, suitable for use in producing a composition of the invention or milk or serum of the invention according to a method of the invention, that is to say, a mixture of endotoxins suitable for inoculating a host animal to produce the desired mixture of antibodies that can be worked up in a suitable manner.

The antigen mixture comprising endotoxins produced by gram negative bacteria is generally injected into an animal to trigger an immunological response to produce the corresponding antibodies. The endotoxins may be sensitised in accordance with accepted laboratory methods and injected in suitable animals under controlled conditions. An adjuvant, for example Freud's Complete Adjuvant, is preferably present in the composition to be injected.

Antibodies prepared in this manner may be type specific e.g. to endotoxins produced by Salmonella enteriditis, or may have a more general specificity, for example to endotoxins produced by many or all gram negative bacteria, or may be a mixture of the above types of antibodies.

The animal to be used for the production of the desired antibodies may be a farm animal, for example a bovine, equine, porcine, ovine, canine or feline animal. The immunisation is, for example, subcutaneous, and the immunisation is generally repeated as often as necessary to maintain·a high level of antibodies in the blood of the immunised animal, which may be used subsequently for the preparation in known manner of the desired antiserum for therapeutic or prophylactic use in the same species of animal or in another species. In the latter case, the serum is preferably subjected to a treatment which generally includes reacting the serum preparation with the blood, blood cells, blood fraction(s) or another tissue preparation of animals of the species to be treated, then removing the added blood or other tissue component(s), for example by centrifugation and/or column chromatography and/or any other procedure.

An antibody composition of the invention preferably comprises a total of not less than 100 micrograms of antibodies per millilitre.

An antibody composition of the invention may be in unit dose or multidose containers, and may be in lyophilised form, for reconstitution before use.

According to one embodiment of this invention, the antibodies specific to the endotoxins may be incorporated in milk or a byproduct thereof. This is particularly useful where antibodies specific to endotoxins are not present naturally in adequate levels in human colostrum and breast milk. Pregnant or lactating cows, mares, ewes, bitches or cats may be immunised with endotoxins to provoke the production of antibodies specific to endotoxins, for example as described above. The milk obtained from such animals contains a high concentration of anti-endotoxin antibodies. This milk or products prepared from this milk may be given to human and animals neonates and children whose mothers' milk is deficient in these antibodies and/or who may benefit from the resulting prophylaxis.

The following Examples illustrate the invention.

Example 1

Cows and other farm animals were immunised by the sub-cutaneous injection of a solution of endotoxins obtained from about 12, for example 11, different bacterial strains and species, which solution was emulsified with an equal volume of Freud's Complete Adjuvant.

The mixture of endotoxins injected above were those obtained from Salmonella enteriditis, Salmonella typhosa, Salmonella typhimurium, Salmonella abortus equii, Eschericia coli 026:B6, Eschericia coli 0128:B12, Eschericia coli 0127:B8, Eschericia coli 055:B5, Salmonella minnesota, Serratia marcescens, and Kleb siella pneumoniae, and, optionally, Shigella flexneri and/or E . coli 0111:B4.

The above composition comprising endotoxins with Freud's Complete Adjuvant was injected as often as required to maintain a high antibody level in milk and/or serum.

a) Milk obtained from the suitably immunised animals that were pregnant or lactating was further processed in the same way as normal milk. The resulting milk, and other products obtained therefrom, was suitable for administering to human and animal neonates and young.

b) Serum which contains a high titre of antibodies specific to the above endotoxins was prepared by bleeding the immunised animal and working up the blood by a method known for the production of an antiserum from blood.

Other bacterial species or strains may be substituted for any one or more of those named above.

Example 2

1. There were obtained compositions of equine, bovine, sheep, canine, feline and porcine serum comprising antibodies which react with and bind endotoxins prepared from the following bacterial sources:

Salmonella typhosa
Salmonella typhimurium
Salmonella abortus equii
Shigella flexneri
Eschericia coli 055:B5
Eschericia coli 0127:B8
Eschericia coli 0128:B12
Eschericia coli 026:B6
Eschericia coli 0111:B4
Salmonella minnesota
Serratia marcescens
Klebsiella pneumoniae

The compositions were prepared as described in Example 1.

2. The total concentration in the composition of all the above anti-endotoxins together was not less than 100 micrograms per millilitre and there was a minimum concentration of 1 microgram per millilitre of at least ten of the about twelve species or strains.

3. The preparation had the ability to kill Klebsiella pneumoniae bacteria in the presence of complement and serum.

4. a) Serum prepared by suitably inoculating a horse was used therapeutically or prophylactically on other horses.

   b) Serum obtained by suitably inoculating a horse was suitably treated to render it fit to use therapeutically or prophylactically on another species of animal, e.g. a cow, sheep, pig, cat or dog.

5. The treatment required in 4b above included reacting the serum preparation with the blood, blood cells, blood fraction(s), or another tissue preparation of animals of the species to be treated, then removing the added blood or other tissue component(s) by centrifugation and/or column chromatography, although any other suitable procedure could be used instead or in addition.

Other strains or species of bacteria may be used instead of or in addition to those listed above, and one or more of the listed sources may be omitted if desired.

Other animals e.g. farm animals may be used for the preparation of serum for therapeutic or prophylactic use in the same or another species of animal, for example as described in 4 and 5 above for horses.

## Claims

1. A composition comprising antibodies produced in response to a mixture of endotoxins obtained from Salmonella enteriditis, Salmonella typhosa, Salmonella typhimurium, Salmonella abortus equii, Escherichia coli 026:B6, Escherichia coli 0128:B12, Escherichia coli 0127:B8, Escherichia coli 055:B5, Salmonella minnesota, Serratia marcescens, and Klebsiella pneumoniae, and, optionally, in addition from Shigella flexneri and/or E. coli 0111:B4; or from Salmonella typhosa, Salmonella typhimurium, Salmonella abortus equii, Shigella flexneri, Escherichia coli 055:B5, Escherichia coli 0127:B8, Escherichia coli 0128:B12, Escherichia coli 026:B6, Escherichia coli 0111:B4, Salmonella minnesota, Serratia marcescens and Klebsiella pneumoniae.

2. A composition according to claim 1 in admixture with a carrier medium, wherein the carrier medium comprises animal or human serum, or physiological saline, or milk or a derivative thereof.

3. A composition according to claim 1 or claim 2, suitable for parenteral or oral administration to a human or an animal.

4. A method of producing a composition as claimed in claim 1, which comprises removing a body fluid comprising a mixture of antibodies obtained from an animal that has been inoculated with a mixture of endotoxins obtained from bacteria as defined in claim 1, and working up the body fluid to give a preparation as claimed in claim 1, suitable for therapeutic and/or prophylactic use in a human and/or animal.

5. A method of producing a mixture of antibodies as claimed in claim 4, wherein the body fluid is blood.

6. A method of producing a mixture of antibodies according to claim 5, wherein the body fluid is milk.

7. A method of producing a composition as claimed in claim 1, which process includes the steps of inoculating a suitable animal with a mixture of endotoxins obtained from bacteria as defined in claim 1, and removing a body fluid containing the resulting mixture of antibodies, with the proviso that no claim is made to the treatment of an animal body by therapy.

8. Milk or serum comprising a mixture of antibodies obtained from an animal that has been inoculated with a mixture of endotoxins obtained from bacteria as defined in claim 1, suitable for use in producing a composition as claimed in claim 1, suitable for therapeutic and/or prophylactic use in a human and/or animal.

9. A mixture of endotoxins obtained from bacteria as defined in claim 1, suitable for use in producing a composition as claimed in any one of claims 1 to 3 or milk or serum as claimed in claim 8, according to a method as claimed in any one of claims 4 to 7.

10. A composition as claimed in any one of claims 1 to 3 for use in the treatment of gram negative bacteremia and endotoxemia.

Claims for the Contracting State: AT

1. A method of producing a composition comprising antibodies produced in response to a mixture of endotoxins obtained from Salmonella enteriditis, Salmonella typhosa, Salmonella typhimurium, Salmonella abortus equii, Escherichia coli 026:B6, Escherichia coli 0128:B12, Escherichia coli 0127:B8, Escherichia coli 055:B5, Salmonella minnesota, Serratia marcescens, and Klebsiella pneumoniae, and, optionally, in addition from Shigella flexneri and or E. coli 0111:B4; or from Salmonella typhosa, Salmonella typhimurium, Salmonella abortus equii, Shigella flexneri, Escherichia coli 055:B5, Escherichia coli 0127:B8, Escherichia coli 0128:B12, Escherichia coli 026:B6, Escherichia coli 0111:B4, Salmonella minnesota, Serratia marcescens and Klebsiella pneumoniae, which comprises removing a body fluid comprising a mixture of antibodies obtained from an animal that has been inoculated with endotoxins obtained from bacteria as defined above, and working up the body fluid to give a composition suitable for therapeutic and or prophylactic use in a human and or animal.

2. A method of producing a mixture of antibodies as claimed in claim 1, wherein the body fluid is blood.

3. A method of producing a mixture of antibodies according to claim 1, wherein the body fluid is milk.

4. A method according to claim 1, wherein the antibodies are admixed with a carrier medium comprising animal or human serum, or physiological saline, or milk or a derivative thereof.

5. A method according to any one of claims 1 to 4, wherein the body fluid is worked up to give a composition suitable for parenteral or oral administration to a human or an animal.

6. A method of producing a composition as claimed in claim 1, which process includes the steps of inoculating a suitable animal with a mixture of endotoxins obtained from bacteria as defined in claim 1, and removing a body fluid containing the resulting mixture of antibodies, with the proviso that no claim is made to the treatment of an animal body by therapy.

7. A method as claimed in claim 6, wherein the body fluid is milk or serum suitable for use in producing a composition as defined in claim 1, suitable for therapeutic and/or prophylactic use in a human and/or animal.

8. Use of a mixture of endotoxins obtained from bacteria as defined in claim 1, for producing a composition as defined in claim 1 or milk or serum as claimed in claim 7, according to a method as claimed in any one of claims 1 to 7.

**Revendications**

1. Composition comprenant des anticorps produits en réponse à un mélange d'endotoxines obtenu à partir de Salmonella enteritidis, Salmonella typhosa, Salmonella typhimurium, Salmonella abortus equii, Escherichia coli 026:B6, Escherichia coli 0120:B12, Escherichia coli 0127:B8, Escherichia coli 055:B5, Salmonella minnesota, Serratia marcescens et Klebsiella pneumoniae et, facultativement, en outre à partir de Shigella flexneri et ou E. coli 0111:B4 ; ou bien à partir de Salmonella typhosa, Salmonella typhimurium, Salmonella abortus equii, Shigella flexneri, Escherichia coli 055:B5, Escherichia coli 0127:B8, Escherichia coli 0128:B12, Escherichia coli 026:B6, Escherichia coli 0111:B4, Salmonella minnesota, Serratia marcescens et Klebsiella pneumoniae.

2. Composition suivant la revendication 1, en mélange avec un milieu servant de support, dans laquelle le milieu servant de support consiste en un sérum d'origine animale ou humaine, ou en sérum physiologique ou bien en lait, ou un de ses dérivés.

3. Composition suivant la revendication 1 ou la revendication 2, convenant pour l'administration parentérale ou orale à un sujet humain ou un animal.

4. Procédé de production d'une composition suivant la revendication 1, qui consiste à prélever un liquide biologique comprenant un mélange d'anticorps obtenu chez un animal auquel a été inoculé un mélange d'endotoxines obtenu à partir de bactéries suivant la revendication 1, et à traiter le liquide biologique pour obtenir une préparation suivant la revendication 1, convenant pour l'utilisation thérapeutique et/ou prophylactique chez un sujet humain et/ou un animal.

5. Procédé de production d'un mélange d'anticorps suivant la revendication 4, dans lequel le liquide biologique est le sang.

6. Procédé de production d'un mélange d'anticorps suivant la revendication 5, dans lequel le liquide biologique est le lait.

7. Procédé de production d'une composition suivant la revendication 1, qui comprend les étapes consistant à inoculer à un animal convenable un mélange d'endotoxines obtenu à partir de bactéries suivant la revendication 1, et à prélever un liquide biologique contenant le mélange résultant d'anticorps, sous réserve qu'aucune revendication ne soit faite en ce qui concerne le traitement du corps d'un animal par des moyens thérapeutiques.

8. Lait ou sérum comprenant un mélange d'anticorps obtenu à partir d'un animal auquel a été inoculé un mélange d'endotoxines obtenu à partir de bactéries suivant la revendication 1, convenant pour l'utilisation dans la production d'une composition suivant la revendication 1, apte à une utilisation thérapeutique et/ou prophylactique chez un sujet humain et/ou un animal.

9. Mélange d'endotoxines obtenu à partir de bactéries suivant la revendication 1, convenant pour l'utilisation dans la production d'une composition suivant l'une quelconque des revendications 1 à 3 ou d'un lait ou d'un sérum suivant la revendication 8, conformément à un procédé suivant l'une quelconque des revendications 4 à 7.

10. Composition suivant l'une quelconque des revendications 1 à 3, destinée à être utilisée dans le traitement de septicémies et/ou d'endotoxémies à bactéries Gram-négatives.

Revendications pour l'Etat contractant suivant: AT

1. Procédé de production d'une composition comprenant des anticorps produits en réponse à un mélange d'endotoxines obtenu à partir de Salmonella enteritidis, Salmonella typhosa, Salmonella typhimurium, Salmonella abortus equii, Escherichia coli 026:B6, Escherichia coli 0128:B12, Escherichia coli 0127:B8, Escherichia coli 055:B5, Salmonella minnesota, Serratia marcescens et Klebsiella pneumoniae, et, facultativement, en outre à partir de Shigella flexneri et/ou E. coli 0111:B4 ; ou bien à partir de Salmonella typhosa, Salmonella typhimurium, Salmonella abortus equii, Shigella flexneri, Escherichia coli 055:B5, Escherichia coli 0127:B8, Escherichia coli 0128:B12, Escherichia coli 026:B6, Escherichia coli 0111:B4, Salmonella minnesota, Serratia marcescens et Klebsiella pneumoniae, qui consiste à prélever un liquide biologique comprenant un mélange d'anticorps obtenu à partir d'un animal auquel ont été inoculées des endotoxines obtenues à partir de bactéries répondant à la définition précitée, et à traiter le liquide biologique pour obtenir une composition convenant pour l'utilisation thérapeutique et/ou prophylactique chez un sujet humain et/ou un animal.

2. Procédé de production d'un mélange d'anticorps suivant la revendication 1, dans lequel le liquide biologique est le sang.

3. Procédé de production d'un mélange d'anticorps suivant la revendication 1, dans lequel le liquide biologique est le lait.

4. Procédé suivant la revendication 1, dans lequel les anticorps sont mélangés à un milieu servant de support, comprenant un sérum d'origine animale ou humaine, ou du sérum physiologique, ou bien du lait ou un de ses dérivés.

5. Procédé suivant l'une quelconque des revendications 1 à 4, dans lequel le liquide biologique est traité pour donner une composition convenant pour l'administration parentérale ou orale à un sujet humain ou un animal.

6. Procédé de production d'une composition suivant la revendication 1, qui comprend les étapes qui consistent à inoculer à un animal convenable un mélange d'endotoxines obtenu à partir de bactéries suivant la revendication 1, et à prélever un liquide biologique contenant le mélange résultant d'anticorps, sous réserve qu'aucune revendication ne soit faite concernant le traitement du corps d'un animal par des moyens thérapeutiques.

7. Procédé suivant la revendication 6, dans lequel le liquide biologique est un lait ou un sérum convenant pour l'utilisation dans la production d'une composition suivant la revendication 1, apte à une utilisation thérapeutique et/ou prophylactique chez un sujet humain et/ou un animal.

8. Utilisation d'un mélange d'endotoxines obtenu à partir de bactéries suivant la revendication 1, pour la production d'une composition suivant la revendication 1, ou bien d'un lait ou d'un sérum suivant la revendication 7, conformément à un procédé suivant l'une quelconque des revendications 1 à 7.

**Ansprüche**

1. Zusammensetzung umfassend Antikörper, die als Reaktion auf eine Mischung von Endotoxinen, erhalten von Salmonella enteriditis, Salmonella typhosa, Salmonella typhimurium, Salmonella abortus equii, Escherichia coli 026:B6, Escherichia coli 0128:B12, Escherichia coli 0127:B8, Escherichia coli 055:B5, Salmonella minnesota, Serratia marcescens und Klebsiella pneumoniae und gegebenenfalls zusätzlich von Shigella flexneri und/oder E. coli 0111:B4; oder von Salmonella typhosa, Salmonella typhimurium, Salmonella abortus equii, Shigella flexneri, Escherichia coli 055:B5, Escherichia coli 0127:B8, Escherichia coli 0128:B12, Escherichia coli 026:B6, Escherichia coli 0111:B4, Salmonella minnesota, Serratia marcescens und Klebsiella pneumoniae.

2. Zusammensetzung nach Anspruch 1 in Mischung mit einem Trägermedium, wobei das Trägermedium tierisches oder menschliches Serum oder physiologische Kochsalzlösung oder Milch oder ein Derivat hievon umfaßt.

3. Zusammensetzung nach Anspruch 1 oder 2, die zur parenteralen oder oralen Verabreichung an einen Menschen oder ein Tier geeignet ist.

4. Verfahren zum Herstellen einer Zusammensetzung nach Anspruch 1, das das Entfernen einer Körperflüssigkeit umfassend eine Antikörpermischung, erhalten von einem Tier, das mit einer Endotoxinmischung, erhalten von Bakterien, wie in Anspruch 1 definiert, angeimpft wurde, und das Aufarbeiten der Körperflüssigkeit zu einer Zusammensetzung nach Anspruch 1, die für therapeutische und oder prophylaktische Verwendung in einem Menschen und oder Tier geeignet ist, umfaßt.

5. Verfahren zum Herstellen einer Antikörpermischung nach Anspruch 4, wobei die Körperflüssigkeit Blut ist.

6. Verfahren zum Herstellen einer Antikörpermischung von Antikörpern nach Anspruch 5, wobei die Körperflüssigkeit Milch ist.

7. Verfahren zum Herstellen einer Zusammensetzung nach Anspruch 1, das die Schritte des Animpfens eines geeigneten Tieres mit einer Mischung von Endotoxinen, erhalten Bakterien, wie in Anspruch 1 definiert, und des Entfernens einer die erhaltene Antikörpermischung enthaltenden Körperflüssigkeit umfaßt, mit der Maßgabe, daß die Behandlung eines Tierkörpers durch Therapie nicht beansprucht wird.

8. Milch oder Serum umfassend eine Antikörpermischung, erhalten von einem Tier, das mit einer Mischung von von den Bakterien, wie in Anspruch 1 definiert, erhaltenen Endotoxinen angeimpft wurde, die bzw. das zur Verwendung beim Herstellen einer Zusammensetzung nach Anspruch 1 geeignet ist, geeignet für therapeutische und/oder prophylaktische Verwendung in einem Menschen und/oder Tier.

9. Mischung von Endotoxinen, erhalten aus Bakterien, wie in Anspruch 1 definiert, die zur Verwendung beim Herstellen einer Zusammensetzung nach einem der Ansprüche 1 bis 3 oder einer Milch oder eines Serums nach Anspruch 8 geeignet ist, gemäß einem Verfahren nach einem der Ansprüche 4 bis 7.

10. Zusammensetzung nach einem der Ansprüche 1 bis 3 zur Verwendung bei der Behandlung Gram-negativer Bakteriämie und Endotoxämie.

Patentansprüche für folgenden Vertragsstaat: AT

1. Verfahren zur Herstellung einer Antikörper enthaltenden Zusammensetzung, die als Reaktion auf eine Mischung von Endotoxinen, erhalten von Salmonella enteriditis, Salmonella typhosa, Salmonella typhimurium, Salmonella abortus equii, Escherichia coli 026:B6, Escherichia coli 0128:012, Escherichia coli 0127:B8, Escherichia coli 055:B5, Salmonella minnesota, Serratia marcescens und Klebsiella pneumoniae und gegebenenfalls zusätzlich von Shigella flexneri und oder E. coli 0111:B4; oder von Salmonella typhosa, Salmonella typhimurium, Salmonella abortus equii, Shigella flexneri, Escherichia coli 055:B5, Escherichia coli 0127:B8, Escherichia coli 0128:B12, Escherichia coli 026:B6, Escherichia coli 0111:B4, Salmonella minnesota, Serratia marcescens und Klebsiella pneumoniae, gebildet wurden, das das Entfernen einer Körperflüssigkeit enthaltend eine Antikörpermischung, erhalten von einem Tier, das mit von Bakterien, wie in Anspruch 1 definiert, erhaltenen Endotoxinen angeimpft wurde, und das Aufarbeiten der Körperflüssigkeit zu einer Zusammensetzung, die für therapeutische und/oder prophylaktische Verwendung in einem Menschen und/oder Tier geeignet ist, umfaßt.

2. Verfahren zum Herstellen einer Antikörpermischung nach Anspruch 1, wobei die Körperflüssigkeit Blut ist.

3. Verfahren zum Herstellen einer Antikörpermischung nach Anspruch 1, wobei die Körperflüssigkeit Milch ist.

4. Verfahren nach Anspruch 1, worin die Antikörper mit einem Trägermedium umfassend tierisches oder menschliches Serum oder physiologische Kochsalzlösung oder Milch oder ein Derivat hievon gemischt werden.

5. Verfahren nach einem der Ansprüche 1 bis 4, worin die Körperflüssigkeit zu einer Zusammensetzung aufgearbeitet wird, die für parenterale oder orale Verabreichung an einen Menschen oder ein Tier geeignet ist.

6. Verfahren zum Herstellen einer Zusammensetzung nach Anspruch 1, das die Schritte des Animpfens eines geeigneten Tieres mit einer Mischung von Endotoxinen, erhalten von Bakterien, wie in Anspruch 1 definiert, und des Entfernens einer die erhaltene Antikörpermischung enthaltenden Körperflüssigkeit umfaßt, mit der Maßgabe, daß die Behandlung eines Tierkörpers durch Therapie nicht beansprucht wird.

7. Verfahren nach Anspruch 6, worin die Körperflüssigkeit Milch oder Serum ist, die bzw. das zur Verwendung beim Herstellen einer Zusammensetzung, wie in Anspruch 1 definiert, geeignet ist, geeignet für therapeutische und/oder prophylaktische Verwendung in einem Menschen und/oder Tier.

8. Verwendung einer Mischung von Endotoxinen, erhalten aus Bakterien, wie in Anspruch 1 definiert, zum Herstellen einer Zusammensetzung, wie in Anspruch 1 definiert, oder von Milch oder Serum, wie in Anspruch 7 beansprucht, gemäß einem Verfahren nach einem der Ansprüche 1 bis 7.